**Europäisches Patentamt**

**European Patent Office**

**Office européen des brevets**

(11) Veröffentlichungsnummer: **0 055 832**
B1

## (12) EUROPÄISCHE PATENTSCHRIFT

(45) Veröffentlichungstag der Patentschrift:
26.02.86

(51) Int. Cl.⁴: **C 12 P 33/02** // C07J71/00

(21) Anmeldenummer: **81110183.1**

(22) Anmeldetag: **05.12.81**

(54) Verfahren zur Herstellung von 11-beta,21-Dihydroxy-2'-methyl-5'-beta-H-1,4-pregnadien-(16,17)-oxazol-3,20-dion.

(30) Priorität: **23.12.80 DE 3049401**

(43) Veröffentlichungstag der Anmeldung:
**14.07.82 Patentblatt 82/28**

(45) Bekanntmachung des Hinweises auf die Patenterteilung:
**26.02.86 Patentblatt 86/9**

(84) Benannte Vertragsstaaten:
**AT BE CH DE FR GB IT LI LU NL SE**

(56) Entgegenhaltungen:
**DE - B - 1 070 176**
**DE - B - 1 093 792**
**GB - A - 908 976**
**US - A - 2 837 464**

**CHEMICAL ABSTRACTS, Band 73, heft 15, 12. Oktober 1970, Seite 104, Zusammenfassung 74110q, COLUMBUS, OHIO (US) M. NAGASAWA et al.: "Microbial transformation of sterols. V. Inhibitors of microbial degradation of cholesterol"**

(73) Patentinhaber: **SCHERING AKTIENGESELLSCHAFT Berlin und Bergkamen,**
**Müllerstrasse 170/178 Postfach 65 03 11,**
**D-1000 Berlin 65 (DE)**

(72) Erfinder: **Weber, Alfred, Dr., Schützallee 56,**
**D-1000 Berlin 37 (DE)**
Erfinder: **Kennecke, Mario, Dr., Taubertstrasse 31 f,**
**D-1000 Berlin 33 (DE)**
Erfinder: **Müller, Rudolf, Dr., Corneliusstrasse 17,**
**D-1000 Berlin 46 (DE)**

## Beschreibung

Die Erfindung betrifft das in den Patentansprüchen gekennzeichnete Verfahren.

Die mikrobiologische $\Delta^1$-Dehydrierung von 11$\beta$,21-Dihydroxy-2'-methyl-5'$\beta$H-4-pregneno (16,17-d)-oxazol-3,20-dion ist, wie eigene Versuche zeigen, nicht so einfach durchführbar, wie diejenige anderer Steroide. Die Fermentation dieser Substanz mit lebenden Kulturen bekannter Steroid-$\Delta^1$-de-hydrierern wie Bacillus lentus ATCC 13 805, Bacillus sphaericus ATCC 7054, ATCC 7055 und ATCC 12 488, Bacillus subtilis NRRL B 558 sowie Norcardia corallina ATCC 4273 und 4275 verläuft unbefriedigend, teils weil kaum eine Umsetzung des Substrates beobachtet wurde, teils weil das Substrat stark metabolisiert wird.

Es wurde nun gefunden, daß die mikrobiologische $\Delta^1$-Dehydrierung von 11$\beta$,21-Dihydroxy-2'-methyl-5'$\beta$H-4-pregneno (16,17-d)-oxazol-3,20-dion gut durchführbar ist, wenn man zur Dehydrierung dieses Substrates einen Mikroorganismus der Spezies Arthrobacter simplex verwendet. Geeignete Arthrobacter simplex Stämme sind beispielsweise die Stämme IFO (3530), ATCC 13260 und insbesondere ATCC 6946.

Es ist seit langem bekannt, daß Arthrobacter simplex — der früher als Corynebacterium simplex bezeichnet wurde — die Tätigkeit zu $\Delta^1$-Dehydrierung von Steroiden besitzt. Er wird in der Technik oft verwendet, da er gegenüber den meisten der obengenannten Steroid-$\Delta^1$-dehydrierern den Vorzug hat, daß die Umsetzung mit diesem Stamm wesentlich rascher abläuft, als mit jenen Mikroorganismen; andererseits hat Arthrobacter simplex aber auch zwei Nachteile:

1. Er neigt oft dazu, bei längerer Fermentationszeit Steroide zu metabolisieren, was zu nicht unerheblichen Ausbeuteverlusten an gewünschtem Verfahrensprodukt führen kann und
2. er neigt oft dazu, die Reaktion zu stoppen, wenn noch 1 bis 3 % Ausgangssteroid in der Kulturbrühe vorhanden ist. Dies erschwert die Herstellung der Verfahrensprodukte in einer Reinheit, wie sie für Arzneimittelwirkstoffe unerläßlich ist, oft ganz erheblich. Die beiden genannten Nachteile werden auch bei der Fermentation von 11$\beta$.21-Dihydroxy-2'-methyl-5'$\beta$H-pregne-no(16,17-d)-oxazol-3,20-dion beobachtet.

Es wurde nun gefunden, daß man diese Nachteile vermeiden kann, wenn man die $\Delta^1$-Dehydrierung mit Arthrobacter simplex in Gegenwart von 0,04 g bis 0,12 g Kobalt(II)-ionen pro Liter Kulturbrühe durchführt. Eine geringere Konzentration an Kobaltionen bringt keinen nennenswerten Effekt, eine höhere Konzentration an Kobalt(II)-ionen wirkt inhibierend. Geeignete Kobalt(II)-ionen liefernde Agenzien sind wasserlösliche Kobaltsalze wie zum Beispiel CoCl$_2$, CoNO$_3$, CoSO$_4$ oder CoSO$_4$, 7H$_2$O. Die Anionen dieser Salze sind für die Durchführbarkeit des Verfahrens ohne Bedeutung, da die Kobalt(II)-salze ja in dem Kulturmedium dissoziieren.

Im übrigen wird das erfindungsgemäße Verfahr unter den Bedingungen durchgeführt, die man üblicherweise zur $\Delta^1$-Dehydrierung von Steroiden mit Mikroorganismen der Spezies Arthrobacter simplex anwendet.

Unter den für diesen Mikroorganismus üblicherweise verwendeten Kulturbedingungen wi in einem geeigneten Nährmedium unter Belüften eine Submerskultur angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Äthanol, Glykolmonomethyläther, Dimethylformamid oder Dimethylsulfoxyd. Ein besonders geeignetes Lösungsmittel ist Hexamethylphosphorsäuretriami Die Emulgierung des Substrats kann beispielsweis bewirkt werden, in dem man dieses in mikronisierter Form oder in einem mit Wasser mischbaren Lösungsmittel (wie Methanol, Äthanol Aceton, Glykolmonomethyläther, Dimethylformam oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxydaddukte oder Fettsäureester von Polyglykolen. Als geeignete Emulgatoren seien die handelsüblichen Netzmittel Tegin[R], Tagat[R], Tween[R] und Span[R] beispielsmäßig genannt.

Die optimale Substratkonzentration, Substratzugabezeit und Fermentationsdauer ist vo der Art des verwendeten Mikroorganismus abhängig. Diese Größen müssen, wie dies bei mikrobiologischen Steroidumwandlungen allgeme erforderlich ist, im Einzelfall durch Vorversuche, w sie dem Fachmann geläufig sind, ermittel werden.

Das nachfolgende Ausführungsbeispiel dient zur Erläuterung des erfindungsgemäßen Verfahrens.

Das in dem nachfolgenden Ausführungsbeispiel verwendete Proteinhydrolysat wurde aus Federme gemäß der Vorschrift von Organic Synthesis, Col. Vol. I, Seite 194 hergestellt, jedoch wurde nach erfolgter Hydrolyse mittels Ammoniak neutralisier und sterilfiltriert. Das erhaltene Filtrat wurde ohne weitere Reinigung als "Proteinhydrolysat" verwendet.

## Beispiel

a. Ein 21-Erlenmeyerkolben mit 1 l steriler Nährlösung enthaltend

     0,5   % Cornsteep liquor
     0,05 % Glucosemonohydrat
     0,1   % Hefeextrakt
     — eingestellt auf pH 7,0 —
wird mit einer Abschwemmung einer Trockenkultu

von Artthrobacter simplex ATCC 6946 beimpft und bei 30° C 72 Stunden mit 180 Umdrehungen pro Minute geschüttelt.

b. Ein 50 l Fermenter mit 30 l steriler Nährlösung enhaltend

    0,5  % Cornsteep liquor
    0,05 % Glucosemonohydrat
    0,1  % Hefeextrakt
    4    ml Silikom SH
    4    ml Pluronic
    — eingestellt auf pH 7,0 —

wird mit 1 l Arthrobacter simplex-Anzuchtskultur beimpft und diese Vorkultur bei 30° C under Belüftung (2 m³ pro Stunde) und Rühren (220 Umdrehungen pro Minute) 24 Stunden lang incubiert.

c. 15 g 11β,21-Dihydroxy-2'-methyl-5'βH-4-pregneno (16,17-d)-oxazol-3,20-dion mit einem Schmelzpunkt von 114/118-130° C werden in 300 ml Hexamethylphosphorsäuretriamid gelöst und anschließend sterilfiltriert.

d. 12 g CoSO$_4$. 7H$_2$O und 12 g (NH$_4$)²SO$_4$ werden in 500 ml Wasser gelöst, auf einen pH-Wert von 6,5 eingestellt und sterilisiert.

e. Ein 50 l-Fermenter wird mit 30 l steriler Nährlösung enthaltend

    0,5  % Cornsteep liquor
    0,05 % Glucosemonohydrat
    0,3  % "Proteinhydrolysat"
    4    ml Silikon SH
    4    ml Pluronic
    — eingestellt auf pH 7,0 —

beschickt, mit 3 l Arthrobacter simplex Vorkultur beimpft und unter Belüften (2 m³ pro Stunde) und Rühren (220 Umdrehungen pro Minute) 6 Stunden lang bei 30° C incubiert. Dann setzt man der Kultur die in Abschnitt c) hergestellte sterile 11β,21-Dihydroxy-2'-methyl-5'βH-4-pregneno (16,17-d)-oxazol-3,20-dion-Lösung und die in Abschnitt d) hergestellten Zusätze zu. Danach wird weiter 20 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50° C unter Vakuum eingeengt. Anschließend erfolgt eine Reinigung durch Chromatographie an Aluminiumoxyd.

man erhält 11,4 g 11β,21-Dihydroxy-2'-methyl-5'βH-1,4-pregnadie-no(16,17-d)-oxazol-3,20-dion vom Schmelzpunkt 210/212-220° C.

## Patentansprüche

Verfahren zur Herstellung von 11β,21-Dihydroxy-2'-methyl-5'βH-1,4-pregnadieno (16,17-d)-oxazol-3,20-dion, dadurch gekennzeichnet, daß man 11β,21-Dihydroxy-2'-methyl-5'βH-pregneno (16,17-d)-oxazol-3,20-dion mit einer lebenden Kultur von Arthrobacter simplex in Gegenwart von 0,04 bis 0,12 g Kobalt(II)-ionen pro Liter Kultur fermentiert.

2. Verfahren zur Herstellung von 11β,21-Dihydroxy-2'-methyl-5'βH-1,4-pregnadie-no(16,17-d)-oxazol-3,20-dion gemäß Anspruch 1, dadurch gekennzeichnet, daß man der Kultur das 11β,21-Dihydroxy-2'-methyl-5'-βH-4-pregneno (16,17-d)-oxazol-3,20-dion als 2 bis 7 gewichtsprozentige Lösung in Hexamethylphosphorsäuretriamid zusetzt.

3. Verfahren zur Herstellung von 11β,21-Dihydroxy-2'-methyl-5'βH-1,4-pregnadieno (16,17-d)-oxazol-3,20-dion gemäß Anspruch 1 bis 2, dadurch gekennzeichnet, daß man die Fermentation mit einer lebenden Kultur von Arthrobacter simplex ATCC 6946 durchführt.

## Claims

1 Process for the preparation of 11β,21-dihydroxy-2'-methyl-5'βH-1,4-pregnadie-no(16,17-d)-oxazole-3,20-dione characterised in that 11β,21-dihydroxy-2'-methyl-5'βH-1,4-pregneno (16,17-d)-oxazole-3,20-dione is fermented with a living culture of Arthrobacter simplex in the presence of 0,04 to 0,12 cobalt(II)ion per litre of culture.

2. Process for the preparation of 11β.21-dihydroxy-2'-methyl-5'βH-1,4-pregnadieno (16,17-d)oxazole-3,20-dione according to claim 1 characterised in that the culture of 11β,21-dihydroxy-2'-methyl-5'βH-1,4-pregneno-(16,17-d)-oxazole-3,20-dione is treated as a 2 to 7 weight per cent solution in hexamethylphosphoric acid triamide.

3. Process for the preparation of 11B.21-dihydroxy-2'-methyl-5'βH-1,4-pregnadieno-(16,17-d)oxazole-3,20-didone according to claim 1 or 2 characterised in that the fermentation is carried out with a living culture of Arthrobacter simplex ATCC 6946.

## Revendications

1. Procédé de préparation de la dihydroxy-11β,21 méthyl-2' H-5'β prégnadiéno-1,4 (16,17-d) oxazole dione-3,20, procédé caractérisé en ce qu'on fait fermenter de la dihydroxy-11β,21 méthyl-2' H-5'β prégnéno-4 (16,17-d) oxazole dione-3,20 avec une culture vivante d'Arthrobacter simplex en présence de 0,04 à 0,12 g d'ions de cobalt(II) par litre de culture.

2. Procédé de préparation de la dihydroxy-11β,21 méthyl-2' h-5'β prégnadiéno-1,4 (16,17-d) oxazole dione-3,20 selon la revendication 1, procédé caractérisé en ce qu'on ajoute à la culture la dihydroxy-11β,21 méthyl-2' H-5'β prégnéno-4 (16,17-d) oxazole dione-3,20 sous la forme d'une solution dans de l'hexaméthylphosphorotriamide dont la concentration est comprise entre 2 et 7% en poids.

3. Procédé de préparation de la dihydroxy-11β,21 méthyl-2' h-5'β prégnadiéno-1,4 (16,17-d) oxazole dione-3,20 selon l'une des revendications 1 et 2, procédé caractérisé en ce qu'on effectue la fermentation avec une culture vivante d'Arthrobacter simplex AYCC 6946.